# EUROPÄISCHE PATENTANMELDUNG

(11) **EP 1 933 153 A1**
(43) Veröffentlichungstag der Anmeldung: **18.06.2008**
(21) Anmeldenummer: 06026094.0
(22) Anmeldetag: 15.12.2006
(51) Int. Cl.: G01N 35/10, G01N 33/543

(54) **Testsystem zur parallelen Durchführung von Multiparameter-Nachweisreaktionen**

(71) Anmelder: Mikrogen Molekularbiologische Entwicklungs-GmbH, 82061 Neuried (DE)
(72) Erfinder: Soutschek, Erwin, 82335 Berg (DE); Motz, Manfred, 80689 München (DE)
(74) Vertreter: Keller, Günter

(57) **Zusammenfassung**

Die vorliegende Erfindung betrifft eine Vorrichtung zur parallelen Durchführung von Multiparameter-Nachweisreaktionen an mehreren Proben. Die erfindungsgemäße Vorrichtung umfasst eine Kartusche in der eine flüssigkeitsdurchlässige Membran befestigt ist. Auf diese Membran kann eine Vielzahl von Antigenen, Antikörpern oder Nukleinsäuren angebracht werden, um den gleichzeitigen Nachweis von verschiedenen Krankheiten in einer Probe zu ermöglichen.

## Beschreibung

Die vorliegende Erfindung betrifft eine Vorrichtung und ein Verfahren zur parallelen Durchführung von Multiparameter-Nachweisreaktionen an mehreren Proben. Mit der erfindungsgemäßen Vorrichtung ist es möglich, ein schnelles und kostengünstiges Diagnoseverfahren zum Nachweis von verschiedenen Krankheiten durchzuführen.

Die Diagnose klinisch-chemischer Parameter wird heutzutage hauptsächlich in Zentrallabors durchgeführt und erfordert kostenintensive Großgeräte. Diese Großgeräte arbeiten jedoch meist nur bei einem hohen Probenaufkommen wirtschaftlich, deshalb lassen kleinere Labors und Krankenhäuser zunehmend ihre diagnostischen Leistungen außer Haus durchführen. Durch das dadurch notwendige Versenden der Proben ist eine patientennahe Sofortdiagnostik zur Abklärung unklarer Symptomatiken nicht möglich.

Ein weiteres Problem bei der Diagnose von Krankheiten ist, dass Krankheitssymptome nicht automatisch Rückschlüsse auf einen einzelnen Erreger zulassen. Bedingt durch die hohen Kosten einer Probenuntersuchung werden daher serologische Parameter oder Tests auf einen bestimmten Krankheitserreger angefordert, die bei gegebener Symptomatik am wahrscheinlichsten zu einer richtigen Diagnose führen können. Leider wird oft nicht auf anhieb die Ursache der Krankheit gefunden und teure Nachuntersuchungen sind die Folge. Um dieses Problem zu lösen, werden Multiparameter-Testsysteme entwickelt, welche die Bestimmung einer Vielzahl von Antikörper-Reaktivitäten oder Krankheitserregern ermöglichen und bei entsprechender Zusammensetzung die Abklärung einer infektionsbedingten Symptomatik sehr schnell und in einem Ansatz gewährleisten.

Neue Testsysteme wie die Luminex-Technologie (www.luminexcorp.com) zum Antikörpernachweis oder der Light-Cycler (www.roche-applied-science.com/lightcycler-online/) auf DNA-Ebene sind bereits bekannt, jedoch aufgrund des apparativen Aufwands und der hohen Kosten nur für Großlabors geeignet.

Ein weiteres Testsystem für ein hohes Probenaufkommen ist aus der CA 02299911 bekannt. Hierin wird die Probenflüssigkeit automatisch aus einem Reagenzglas in ein Testbehältnis gefüllt und dort mit verschiedenen Reagenzien versetzt. Anschließend wird das Reaktionsgemisch auf eine ELISA-Immunoassay-Membran aufgetragen und der gewünschte Test durchgeführt. Beim Auftragen des Reaktionsgemisches wird ein Filter verwendet, der wieder entfernt werden muss, bevor das Testergebnis mittels eines bildgebenden Verfahrens ermittelt wird. Das in der CA 02299911 beschriebene Testsystem stellt jedoch kein kostengünstiges System für ein kleines Probenaufkommen dar und weist einen teuren und mechanisch komplexen Aufbau auf.

Im Gegensatz dazu ist ein kleineres Testsystem zur Bestimmung von Mikroorganismen aus der EP 0 537 827 bekannt. Darin wird ein Verfahren beschrieben, in welchem eine Probenlösung in einen Behälter eingefüllt wird, der durch eine mikroporöse Membran in ein oberes und ein unteres Kompartiment geteilt wird. Auf der Oberfläche der Membran befinden sich Antigene von Mikroorganismen, welche an die Antikörper aus der Probenlösung binden können. Die Probenlösung fließt nach dem Einbringen in den Behälter durch die Membran in das untere Kompartiment, in dem sich ein absorbierendes Material befindet. Die Fließgeschwindigkeit der Probenlösung durch die Membran wird in der EP 0 537 827 durch die Porengröße der Membran, die Dichte oder Hydrophilie des absorbierenden Materials bestimmt.

Nachteilig bei diesem Verfahren ist jedoch, dass das automatische Auswerten der Testergebnisse mittels eines Scanners durch die Anordnung der Membran in dem Behälter nachteilig beeinflusst wird. Des Weiteren ist die nachträgliche Beeinflussung der Fließgeschwindigkeit nach Wahl der Porengröße der Membran und dem absorbierbaren Mittel nicht mehr möglich. Zudem ist das Fassungsvolumen an Probenlösung durch das absorbierende Mittel und das Fassungsvermögen des unteren Kompartiments beschränkt. In der EP 0 537 827 wird zwar die Verwendung eines Vakuums zum Durchsaugen der Probenlösung durch die Membran angedeutet, welches das Absaugen der Probenlösung aus dem unteren Kompartiment ermöglichen würde, jedoch offenbart die Patentschrift keine einfache und schnell zu bedienende Vorrichtung für einen derartigen Zweck.

Ein weiteres Testsystem ist aus dem US-Patent 4,948,561 bekannt. Hierin wird ein Probengefäß mit einer Probenlösung befüllt und anschließend mit einer Filtervorrichtung verschlossen, die eine Membran zur Durchführung von Immunoassays enthält. Das geschlossene System aus Probenbehälter und Filtervorrichtung ermöglicht jedoch kein nachträgliches Auftragen von weiteren Wasch- und Reaktionslösungen. Des Weiteren kann ein derartiges System nicht mit einer Vakuumpumpe zum Durchsaugen von größeren Mengen an Probenlösung betrieben werden.

Aus der EP 0 444 303 ist ein Verfahren zur Detektierung von Chlamydia Antigenen beschrieben, worin eine Probe durch eine mikroporöse Membran fließt, welche Anti-Chlamydia Antikörper auf der Oberfläche enthält. EP 0 444 303 offenbart jedoch keine Vorrichtung zur einfachen und kostengünstigen Durchführung eines Multiparameter-Nachweistests von Proben.

Wie an den bekannten Testsystemen deutlich wird, gibt es bisher keine Vorrichtung, die ein kostengünstiges, schnelles und einfaches Durchführen von Multiparameter-Nachweisreaktionen ermöglicht. Wie oben beschrieben, sind zwar großtechnische Geräte für ein hohes Probenaufkommen, sowie Testsysteme für einzelne Proben bekannt, jedoch ist es für Arztpraxen und Krankenhäuser sehr interessant, ein Testsystem für beispielsweise drei bis zehn Proben zur Verfügung zu haben, das nicht kontinuierlich betrieben werden muss, jedoch bei Bedarf eine schnelle Diagnose erlaubt. Besonders vorteilhaft ist hierbei eine einfache Handhabung, die keine Laborausbildung erfordert und durch eingewiesenes Personal durchgeführt werden kann.

Des Weiteren sollten die Teile der Vorrichtung, z.B. der Probenbehälter mit der Membran, geringe Herstellungs- und Materialkosten aufweisen. Weiterhin wünschenswert ist eine verkürzte Inkubationszeit der einzelnen Reagenzien, welche ein schnelles Testergebnis ermöglicht. Um die Gesamtdauer für die Durchführung der Multiparameter-Nachweisreaktionen zu verkürzen, wäre es vorteilhaft, notwendige Waschschritte bei hoher Fließgeschwindigkeit durchführen zu können. Dies bringt hohe Anforderungen für die verwendete Membran mit sich, da gewöhnliche Membranmaterialien einem stark erhöhten Druck nicht standhalten.

Die oben beschriebenen Probleme der bekannten Testsysteme werden durch die vorliegende Vorrichtung gelöst. Die erfindungsgemäße Vorrichtung umfasst einmal-verwendbare Kartuschen, die auf einfache und schnelle Weise in die dazu vorgesehene Kartuschenhalterung eingesetzt werden können, so dass die Kartusche und die Kartuschenhalterung flüssigkeitsdicht verbunden sind. Die Kartuschen sind mit Kaminen versehen, die als Probenbehälter zum Einfüllen der Probenlösung dienen. Diese Kamine können nach Durchführung des Testverfahrens wieder schnell entfernt werden, um ein effizientes und genaues Auslesen des Testergebnisses, entweder visuell oder mit einem integrierten Scanner, zu ermöglichen. Die vorliegende Erfindung stellt zudem eine Membran bereit, die durch ein geeignetes Unterstützungsmittel, beispielsweise ein Filterpapier verstärkt wurde und dadurch der hohen Fließgeschwindigkeit standhält und eine kurze Gesamtdauer für das gesamte Testverfahren ermöglicht. Vorteilhaft ist auch, dass die Fließgeschwindigkeit der Lösungen durch die Membran über die Vakuumpumpe gesteuert werden kann.

Ein weiterer Vorteil der erfindungsgemäßen Vorrichtung bzw. des Verfahrens entsteht dadurch, dass mehrere unterschiedliche Sonden, beispielsweise Antigene, Antikörper oder Nucleinsäuren, für dieselbe Krankheit auf die flüssigkeitsdurchlässige Membran räumlich getrennt voneinander aufgetragen werden können, um beispielsweise eine Differenzierung des Infektionsablaufs oder des Serotyps zu erreichen. Besonders interessant ist dies bei Krankheiten, hervorgerufen durch den Epstein-Barr-Virus (EBV), Cytomegalovirus (CMV) oder Herpes Simplex-Virus (HSV). Die EBV Serologie hat wegen der Vielgestaltigkeit des klinischen Bildes der EBV Primärinfektion eine große differentialdiagnostische Bedeutung. Die bei der infektiösen Mononukleose zu beobachtende Lymphadenopathie kann mit Symptomen verwechselt werden, die durch den Rötelnvirus, Mumpsvirus, Zytomegalievirus oder eine HIV-Infektion, mit Toxoplasmose, Brucellose und Leptospirose hervorgerufen werden. Die erhöhten Leberenzymwerte müssen von Infektionen mit Hepatitiserregern wie Hepatitis-A, Hepatitis-B und Hepatitis-C abgegrenzt werden. Daher ist es sehr vorteilhaft, dass die erfindungsgemäße Vorrichtung die Untersuchung auf all diese Erreger gleichzeitig ermöglicht.

Ein weiterer Vorteil der vorliegenden Erfindung liegt in der Möglichkeit, die Testergebnisse mittels eines Scanners automatisch auszulesen. Aufgrund der begrenzten Tiefenschärfe eines Scanners, sind hierfür nur die erfindungsgemäßen Vorrichtungen geeignet. Durch die Möglichkeit, den Kamin abzunehmen oder den Kamin trichterförmig auszuformen, ist der Abstand zwischen Membran und Kaminoberseite, trotz ausreichend großen Volumens, nicht zu groß.

Zudem weist die erfindungsgemäße Vorrichtung eine flüssigkeitsdurchlässige Membran auf, die Teststellen für Kontrollzwecke, bspw. Negativ- bzw. Positivkontrollen umfasst. Diese Kontrollzwecke können beispielsweise Färbekontrollen für einen korrekten Testablauf, die Anzeige des verwendeten Konjugats oder cutoff-Kontrollen für die Berechnung der relativen Intensitäten der gefärbten Stellen (nach Durchführung der Nachweisreaktionen) auf der Membranoberfläche sein. Die erfindungsgemäßen Teststellen für Kontrollzwecke auf der Membranoberfläche ermöglichen eine exaktere Auswertung der Testergebnisse, beispielsweise unter Verwendung eines Scanner-Systems.

Die erfindungsgemäße Vorrichtung ist weiterhin vorteilhaft bei der kostengünstigen, einfachen Schnelldiagnose von vielen Erkrankungen, wie etwa einer Lungenentzündung. Dabei ist es notwendig, den Erreger direkt nachzuweisen, um eine schnelle und effektive Behandlung des Patienten zu gewährleisten. Hier muss prinzipiell zwischen bakteriellen und viralen Infektionen unterschieden werden. Zudem ist die zweifelsfreie Bestimmung des Erregers, bei dem einen Vielzahl von Vertretern beider Erregergruppen in Frage kommen, entscheidend für die Therapie. Die gängigen Formate in der Serologie zum Erregernachweis auf der Basis von Antigen-/ Antikörperreaktionen (z.B. ELISA-Test) sind nicht geeignet, eine Differentialdiagnostik mit geringem technischen Aufwand, kostengünstig und schnell durchzuführen. So wird bei den ELISA-Tests ein hoher Automatisierungsgrad mit aufwendigem Laborequipment oder eine arbeitsintensive Einzeldiagnostik benötigt, und man hat, ebenso wenig wie bei Line-Tests, nicht die Möglichkeit viele Parameter durch verschiedene Sonden nachzuweisen.

Um eine größere Anzahl von unterschiedlichen Testsonden auf eine Membran aufzubringen, um diese dann auch gegebenenfalls visuell auszuwerten, muss das Testsystem für eine ausreichend große Membran und Probenmenge geeignet sein. Ein Testsystem, das all diese Anforderungen erfüllt, ist nicht bekannt.

Die vorliegende Erfindung betrifft eine Vorrichtung zur parallelen Durchführung von Multiparameter-Nachweisreaktionen an mehreren Proben, umfassend:
a) mindestens eine Kartusche (1),
b) je Kartusche (1) eine darin befestigte, flüssigkeitsdurchlässige Membran (2), worin die flüssigkeitsdurchlässige Membran (2) an der unteren Seite bevorzugt mit einem Filterpapier verstärkt ist,
c) einen Kamin (4), der auf der Kartusche so befestigt wird, dass ein nach oben offenes, oberes Kompartiment erzeugt wird, und
d) auf der ins obere Kompartiment weisenden, oberen Oberfläche der flüssigkeitsdurchlässigen Membran (2) angebrachte Sonden, die den gleichzeitigen Nachweis von verschiedenen Krankheiten in einer Probe ermöglichen,
e) eine Kartuschenhalterung (5) für die Kartuschen (1), welche das Absaugen von Flüssigkeiten aus dem oberen Kompartiment durch die flüssigkeitsdurchlässige Membran (2) ermöglicht, und
f) Mittel zum Erzeugen eines Vakuums an der Unterseite der flüssigkeitsdurchlässigen Membran (2).

In einer besonderen Ausführungsform der Vorrichtung weist die flüssigkeitsdurchlässige Membran (2) einen Durchmesser zwischen 1 cm und 10 cm, weiter bevorzugt zwischen 1 cm und 7 cm, noch weiter bevorzugt zwischen 1 cm und 4 cm und am meisten bevorzugt zwischen 1,5 cm und 2,5 cm auf.

In einer weiter bevorzugten Ausführungsform der Vorrichtung besteht die flüssigkeitsdurchlässige Membran (2) aus einem Material, ausgewählt aus der Gruppe bestehend aus: Nitrozellulose, Polycarbonat, Polyamid, Polyester, Polyvinylidenfluorid und Nylon. Weiter bevorzugt besteht die flüssigkeitsdurchlässige Membran (2) aus Nitrozellulose.

In einer bevorzugten Ausführungsform der Vorrichtung kann das obere Kompartiment ein Volumen von 1 - 8 ml, bevorzugt 6 ml, aufnehmen.

In einer anderen Ausführungsform weist die flüssigkeitsdurchlässige Membran (2), die mit einem Filterpapier (6) verstärkt ist, eine Dicke von 0,5 - 1,5 mm, bevorzugt 1 mm, auf.

Die besonders bevorzugte Porengröße der flüssigkeitsdurchlässigen Membran (2) liegt zwischen 0,2 - 0,8 µm.

Eine weiter bevorzugte Ausführungsform der Vorrichtung umfasst einen Scanner (10) zum optischen Auslesen der Testergebnissen.

Bevorzugt ist, dass die Kartusche (1) der erfindungsgemäßen Vorrichtung durch eine Drehbewegung in die Kartuschenhalterung (5) eingebracht wird. Weiter bevorzugt ist, dass die Kartusche zur Steckrichtung senkrechte längliche Erhebungen aufweist.

In einer bevorzugten Ausführungsform der Vorrichtung werden unterschiedliche Sonden an voneinander räumlich getrennten Stellen auf der flüssigkeitsdurchlässigen Membran (2) angebracht.

In einer noch bevorzugteren Ausführungsform der Vorrichtung werden mehrere Arten von Sonden für dieselbe Krankheit auf die flüssigkeitsdurchlässige Membran (2) räumlich getrennt voneinander aufgetragen. Besonders bevorzugt werden hierbei mindestens zwei Arten von Sonden für den gleichen Krankheitserreger verwendet, wobei der Krankheitserreger ausgewählt aus der Gruppe bestehend aus: Epstein-Barr-Virus (EBV), Cytomegalovirus (CMV) Herpes Simplex-Virus (HSV), sexuell übertragbaren Krankheiten (STD), Pneumonien und Autoimmunkrankheiten.

In einer weiter bevorzugten Ausführungsform sind die Sonden auf der flüssigkeitsdurchlässigen Membran (2) so ausgewählt, dass sie entweder zur Abklärung einer unklaren Symptomatik (z.B. Zustand mit unklarem Fieber, Lymphknotenschwellung, generelles Unwohlsein), bei respiratorischen Infekten oder zum Abklären von rheumatischen/arthritischen Beschwerden oder einer mykobakteriellen Infektion geeignet sind. Bei dem Abklären einer unklaren Symptomatik sind Sonden bevorzugt geeignet, die Antigene für Krankheitserreger darstellen, ausgewählt aus der Gruppe bestehend aus: Epstein-Barr-Virus (EBV), Herpes simplex-Virus (HSV), Cytomegalovirus (CMV), Varicella Zoster Virus (VZV), Parvovirus B19, Borrelia burgdorferi und Treponema pallidum. Die Sonden zum Abklären von rheumatischen/arthritischen Beschwerden umfassen Antigene aus der Autoimmun-Diagnostik (ANA/ENA) und/ oder Antigene für mindestens einen Krankheitserreger ausgewählt aus der Gruppe bestehend aus Parvovirus B19, Cytomegalovirus (CMV), Epstein-Barr-Virus (EBV), Yersinia enterocolitica, Campylobacter.

In einer besonders bevorzugten Ausführungsform werden mindestens 4, weiter bevorzugt mindestens 8 Sonden für den Nachweis von Krankheitserregern verwendet, die ausgewählt sind aus der Gruppe bestehend aus: EBV, CMV, HSV, CMV, VZV, Parvovirus B19, Borrelia burgdorferi und Treponema pallidum.

In einer weiteren Ausführungsform der erfindungsgemäßen Vorrichtung weist die Kartusche (1) eine seitliche Haltevorrichtung (3) als Mittel zum Greifen der Kartusche während der Drehbewegung beim Einsetzen der Kartusche (1) in die Kartuschenhalterung (5) auf. Besonders bevorzugt wird die Kartusche (1) durch eine Drehbewegung in der Kartuschenhalterung (5) befestigt.

In einer weiter bevorzugten Ausführungsform der Vorrichtung wird die Probenflüssigkeit mittels einer Pumpe mit einer Fließgeschwindigkeit von 0,1 - 2 ml/min durch die flüssigkeitsdurchlässige Membran (2) gesaugt. Es ist weiter bevorzugt, dass die Waschlösung mit einer Fließgeschwindigkeit von 2-10 ml/min, weiter bevorzugt 4-10 ml/min durch die flüssigkeitsdurchlässige Membran (2) gesaugt wird.

Des Weiteren betrifft die vorliegende Erfindung ein Verfahren zur parallelen Durchführung von Multiparameter-Nachweisreaktionen an mehreren Proben, wobei jedes Testsystem, bevorzugterweise jedoch die erfindungsgemäße Vorrichtung verwendet werden kann, umfassend folgende Schritte:
a) Einfüllen der Probenflüssigkeiten in den Kamin der Kartuschen,
b) Anlegen eines Unterdrucks an die Unterseite der flüssigkeitsdurchlässigen Membran der Kartusche zum Durchsaugen der Probenflüssigkeit durch die flüssigkeitsdurchlässige Membran,
c) mindestens zwei Waschschritte, bei denen mittels Vakuum eine Waschlösung durch die flüssigkeitsdurchlässige Membran gesaugt der Spüllösung durch die Membran,
d) Abnehmen der Kamine der Kartuschen,
e) Auslesen der Testergebnisse mit einem Scanner oder visuell, bevorzugt mit einem Scanner.

Bevorzugt ist, dass zu Beginn des Verfahrens mindestens zwei Kartuschen (1) durch eine seitliche Drehbewegung in die Kartuschenhalterung (5) eingebracht werden.

Weiter bevorzugt ist ein Verfahren, worin die Probenflüssigkeit mittels einer Pumpe mit einer Fließgeschwindigkeit von 0,1-2 ml/min durch die flüssigkeitsdurchlässige Membran (2) gesaugt. Es ist weiter bevorzugt, dass die Waschlösung mit einer Fließgeschwindigkeit von 2 - 10 ml/min, weiter bevorzugt 4 - 10 ml/min durch die flüssigkeitsdurchlässige Membran (2) gesaugt wird. Besonders bevorzugt ist, dass alle Kartuschen (1) mit einer Pumpe (8) in Verbindung stehen.

In einer noch bevorzugteren Ausführungsform erfolgt das visuelle Auslesen mit Hilfe von Schablonen, die nach dem Abnehmen der Kamine auf die Kartuschen gelegt werden können und die Zuordnung der Stellen mit Sonden erlaubt.

Im Nachfolgenden wird die erfindungsgemäße Vorrichtung im Detail beschrieben. Alle Ausführungsformen der erfindungsgemäßen Vorrichtung sind zur Durchführung des erfindungsgemäßen Verfahrens geeignet.

Die erfindungsgemäße Vorrichtung umfasst mindestens zwei Kartuschen, weiter bevorzugt mindestens drei, noch weiter bevorzugt mindestens vier, weiter bevorzugt mindestens fünf und am meisten bevorzugt mindestens sechs Kartuschen. Diese Kartuschen können aus jedem Material gefertigt sein, wobei die Kartuschen bevorzugt aus Kunststoff gefertigt sind, da die Kartuschen nach einmaligem Gebrauch entsorgt werden. Besonders bevorzugt sind Kunststoffe mit geringer Neigung zur Proteinbindung. Die Kartusche enthält die Membran, auf deren Oberfläche die Nachweisreaktionen durchgeführt werden.

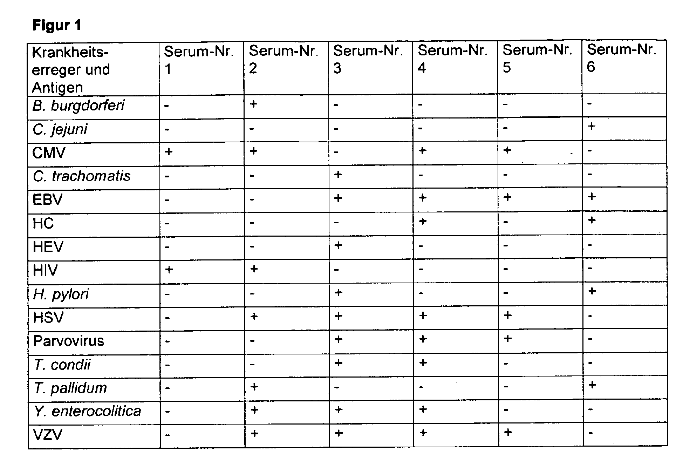

Die Kartusche kann in jeder beliebigen Form ausgebildet sein, bevorzugt ist sie jedoch röhrenförmig ausgebildet, wobei der Röhrenquerschnitt mindestens den Durchmesser der Membran aufweist, (siehe Figur 1). Die Membran ist mit der Kartusche flüssigkeitsdicht verbunden, so dass die Probenflüssigkeit später ausschließlich durch die Membran hindurchfließt, jedoch nicht seitlich an der Membran vorbei. Die Aufgabe der Kartusche ist es, die Membran mit dem Kamin und der Kartuschenhalterung zu verbinden. Da die Kartusche selbst nicht den Behälter für die Probenlösung bildet, ist es bevorzugt, dass sich die Kartusche in der Höhe (senkrecht zur Membran) nicht weit über die Membran hinaus ausdehnt, besonders bevorzugt hat die Kartusche eine Höhe von 1-4 cm, noch bevorzugter 1-3 cm, am meisten bevorzugt 1 - 2 cm.

Zusätzlich kann an der Kartusche ein seitliche Haltevorrichtung angebracht sein, die als Mittel zum Festhalten der Kartusche dient, wenn beispielsweise bei einem Drehverschlussmechanismus die Kartusche durch eine Drehbewegung in die Kartuschenhalterung eingesetzt wird.

Auf die Kartusche der erfindungsgemäßen Vorrichtung ist ein Kamin fest oder abnehmbar angebracht, der ein nach oben offenes Kompartiment zur Verfügung stellt. Das nach oben offene Kompartiment dient als Probenbehälter, in den die Probenlösung später eingebracht werden kann. Das nach oben offene Kompartiment wird seitlich durch den Kamin und an der Unterseite durch die Kartusche bzw. durch die flüssigkeitsdurchlässige Membran abgeschlossen.

Der Kamin kann in jeder Form ausgebildet sein, bevorzugt orientiert sich seine Form an der Kartusche oder der flüssigkeitsdurchlässigen Membran, weiter bevorzugt an der Form der flüssigkeitsdurchlässigen Membran, wobei der Kamin mit der Kartusche, auf der er angebracht wird, flüssigkeitsdicht verbunden ist. Bevorzugterweise ist die flüssigkeitsdurchlässige Membran kreisrund, so dass auch der Kamin in Form einer Röhre ausgeformt werden kann. Es ist aber auch möglich, den Kamin in jeglicher anderer Form auszuformen, solange er mit der Kartusche bündig abschließt. In einer anderen bevorzugten Ausführungsform der Erfindung ist der Kamin trichterförmig ausgeformt, um den Abstand zwischen Membran und Kaminoberseite, trotz großen Volumens gering zu halten.

Der Kamin kann aus jedem Material gefertigt sein, bevorzugt wird er jedoch wie die Kartusche aus Kunststoff hergestellt. Die Höhe des Kamins (senkrecht zur Membran) orientiert sich an dem gewünschten Fassungsvermögen des nach oben offenen Kompartiments. Bevorzugterweise beträgt die Höhe des Kamins 1 - 10 cm, weiter bevorzugt 1 - 6 cm, noch weiter bevorzugt 1 - 3 cm.

In einer Ausführungsform der Erfindung kann der Kamin nach Durchführung der Nachweisreaktionen wieder von der Kartusche entfernt werden, um eine leichtere Auswertung des Testergebnisses zu ermöglichen. Der Kamin kann durch jeden Mechanismus mit der Kartusche verbunden werden, wobei der Fachmann geeignete Verschluss- oder Aufsteckmechanismen dazu kennt. Bevorzugt weist die Kartusche einen äußeren Durchmesser auf, der mit dem inneren Durchmesser des Kamins übereinstimmt, so dass der Kamin auf die Kartusche aufgesteckt werden kann. Bevorzugterweise ist der Kamin derart mit der Kartusche verbunden, dass der Kamin abgenommen werden kann, ohne dass sich die Kartusche aus der Kartuschenhalterung löst. Bevorzugt wird das Abnehmen des Kamins durch eine Dreh-/ Ziehbewegung erreicht, wobei die Ziehbewegung von der Kartuschenhalterung weg und die Drehrichtung in die Richtung zeigt, in der die Kartusche in der Kartuschenhalterung befestigt wird.

Des Weiteren umfasst die erfindungsgemäße Vorrichtung eine Kartuschenhalterung in der die Kartuschen befestigt werden. Die Kartuschenhalterung kann frei auf dem Untergrund stehen oder auf einer Trägerplatte montiert sein. Die Kartuschen können in die Kartuschenhalterung eingebracht werden, unabhängig davon, ob der Kamin bereits an der Kartusche befestigt ist, oder dies erst später passiert. Die Kartusche kann durch jeden Verschlussmechanismus mit der Kartuschenhalterung verbunden werden, wobei ein Fachmann geeignete Mittel dafür kennt. Die Kartusche und die Kartuschenhalterung werden flüssigkeitsdicht miteinander verbunden, damit die Probenflüssigkeit bzw. eventuelle Wasch- und Reaktionslösungen durch die flüssigkeitsdurchlässige Membran in die Kartuschenhalterung überführt werden kann. Bevorzugterweise wird die Kartusche durch einen Bajonett-Verschluss in der Kartuschenhalterung befestigt. Hierbei wird die Kartusche durch eine Steck-Dreh-Bewegung mit der Kartuschenhalterung verbunden. Dazu werden die beiden zu verbindenden Teile ineinander gesetzt, wobei in beiden Teilen annähernd senkrecht zur Steckrichtung an der Verbindungsstelle längliche Erhebungen (Mittel zum Befestigen in der Kartuschenhalterung) angebracht sind. Diese laufen dennoch nicht rundum, sondern sind unterbrochen, sonst wäre das Ineinanderstecken nicht möglich. Da die Erhebungen nun leicht schräg in der Ebene senkrecht zur Steckrichtung liegen, werden durch eine Drehbewegung beide Teile gegeneinander gepresst. Der Bajonett-Verschluss arbeitet auf diese Art und Weise wie ein Gewinde. Bevorzugt haben die seitlichen Erhebungen der Kartusche die Form von kleinen Flügeln, die durch die Eindrehbewegung in die Kartuschenhalterung eingeklemmt werden. Durch diesen Verschluss wird eine luftdichte Verbindung zwischen Kartusche und Kartuschenhalterung ermöglicht. Wie oben erwähnt, sind jedoch auch andere Verschlussmechanismen möglich.

Durch das Verbinden der Kartusche mit der Kartuschenhalterung entsteht ein unteres Kompartiment, auf der dem oberen Kompartiment gegenüberliegenden Seite. Dieses untere Kompartiment wird von der Unterseite der flüssigkeitsdurchlässigen Membran bzw. der Kartusche und der Kartuschenhalterung abgegrenzt. Des Weiteren kann an die Kartuschenhalterung bevorzugterweise ein Anschluss für einen Schlauch angebracht werden oder ist in die Kartuschenhalterung integriert. Dadurch entsteht durch das Befestigen der Kartusche in der Kartuschenhalterung eine Verbindung zwischen der Pumpe und der Kartusche bzw. durch die Membran hindurch mit dem oberen Kompartiment.

Das untere Kompartiment kann in jeglicher Form ausgebildet sein. Bevorzugterweise ist das Durchlaufen der Probenflüssigkeit durch die flüssigkeitsdurchlässige Membran an jeder mit Sonden versehenen Stelle möglich. Bevorzugterweise ist das untere Kompartiment trichterförmig ausgebildet und verjüngt sich in der von der flüssigkeitsdurchlässigen Membran wegweisenden Richtung. Das untere Kompartiment ist bevorzugt nach unten oder zur Seite hin offen und kann an eine Schlauchverbindung angeschlossen werden. Diese Anordnung ermöglicht es, dass die Probenflüssigkeit aus dem oberen Kompartiment durch die flüssigkeitsdurchlässige Membran in das untere Kompartiment gelangt und von dort abgesaugt wird.

In einer anderen Ausführungsform der Vorrichtung erstreckt sich die Kartusche auf der von dem Kamin abgewandten Seite über die flüssigkeitsdurchlässige Membran hinaus und bildet ein nach unten offenes Kompartiment. Zu diesem Kompartiment kann dann beispielsweise über einen Schlauch eine Verbindung zu der Pumpe hergestellt werden. Durch Befestigen der Kartusche in der Kartuschenhalterung wird dann ebenfalls eine flüssigkeitsdichte Verbindung zu der Pumpe hergestellt.

Die Kartuschenhalterung stellt Plätze für mehrere Kartuschen bereit. Bevorzugterweise enthält die Kartuschenhalterung Platz für mindestens zwei Kartuschen, weiter bevorzugt drei, weiter bevorzugt vier, noch weiter bevorzugt fünf und am meisten bevorzugt sechs Kartuschen. Zum Absaugen der Probenflüssigkeit aus dem oberen Kompartiment bzw. zum Erzeugen eines Vakuums im unteren Kompartiment wird bei der erfindungsgemäßen Vorrichtung eine Pumpe verwendet. Für diese Verwendung geeignete Pumpen sind dem Fachmann bekannt und er ist in der Lage, eine Pumpe mit einer geeigneten Leistung auszuwählen. Bevorzugterweise wird eine Peristaltikpumpe verwendet, die die unten spezifizierten Fließgeschwindigkeiten, beim Absaugen der Probenlösung bzw. der Wasch- und Reaktionslösungen, unter Berücksichtigung der Anzahl der verwendeten Kartuschen, erzeugen kann. Aber auch eine Wasserstrahlpumpe kann bei einfachen Ausführungsformen genügen.

Die Pumpe steht bevorzugterweise über Schläuche mit den verschiedenen Kartuschen in Verbindung. Ein Ende eines Schlauches ist mit der Pumpe verbunden, das andere Ende ist mit der Kartuschenhalterung verbunden. Die abgesaugte Probenflüssigkeit bzw. eventuelle Wasch- und Reaktionslösungen können bevorzugterweise in einem gemeinsamen Entsorgungsbehälter gesammelt werden.

Zusätzlich ist bevorzugt, dass Mittel zur Steuerung der Pumpe mittels geeigneter Software verwendet werden. Durch Variierung der Pumpenleistung ist es möglich, dass die Fließgeschwindigkeit für das Durchsaugen der Probenlösung bzw. der Wasch- und Reaktionslösung gesteuert wird.

Um die erfindungsgemäßen Fließgeschwindigkeit zu ermöglichen, wird die flüssigkeitsdurchlässige Membran bevorzugt mit einem Filterpapier verstärkt. Eine bevorzugte flüssigkeitsdurchlässige Membran mit Filterpapier ist von der Firma Schleicher & Schüll erhältlich, z.B. mit der Bezeichnung FT 060. Durch Einstellen der Pumpenleistung kann dadurch die geeignete Fließgeschwindigkeit der Probenlösung bzw. der Wasch- und Reaktionslösungen durch die flüssigkeitsdurchlässige Membran gewählt werden. Des weiteren ist bevorzugt, dass die Probenflüssigkeit langsamer durch die flüssigkeitsdurchlässige Membran gesaugt wird als die Waschlösungen bzw. Reaktionslösungen. Die notwendige Pumpenleistung am unteren Kompartiment ist von der Zahl der Kartuschen, dem Durchmesser der flüssigkeitsdurchlässigen Membran, deren Porengröße, dem Filterpapier zur Verstärkung der Membran usw. abhängig. Die Pumpenleistung wird so gewählt, dass eine ausreichende Inkubationszeit der Probenflüssigkeit mit den Antigenen, Antikörpern oder Nukleinsäuren gewährleistet ist. In einer weiter bevorzugten Ausführungsform der Vorrichtung wird die Probenflüssigkeit mittels einer Pumpe mit einer Fließgeschwindigkeit von 0,1 - 2 ml/min, weiter bevorzugt 0,5 - 2 ml/min und noch weiter bevorzugt 1 - 2 ml/min und am meisten bevorzugt 1,5 - 2 ml/min durch die flüssigkeitsdurchlässige Membran gesaugt. Es ist weiter bevorzugt, dass die Waschlösung mit einer Fließgeschwindigkeit von 2 - 10 ml/min, weiter bevorzugt mit 4 - 10 ml/min, noch weiter bevorzugt mit 6 - 10 ml/min und am meisten bevorzugt mit 8 - 10 ml/min durch die flüssigkeitsdurchlässige Membran gesaugt wird. Hierbei können die Fließgeschwindigkeiten für die Probenlösung, die Waschlösung und die Reaktionslösungen unabhängig voneinander bestimmt werden.

Die flüssigkeitsdurchlässige Membran, die in der erfindungsgemäßen Kartusche befestigt ist, kann aus jedem Material gefertigt sein, welches das Anbringen von Antigenen, Antikörpern oder Nukleinsäuren erlaubt, welches für die Probenlösung durchlässig ist und Poren in einer geeigneten Größe aufweist. Üblicherweise bewegen sich Porengrößen von Membranen in einem Bereich zwischen 0,2 und 0,8 µm. Bevorzugt wird in der erfindungsgemäßen Vorrichtung eine Porengröße zwischen 0,5 und 0,7 µm, weiter bevorzugt mit 0,6 µm, verwendet.

Geeignete Materialien für die Membran sind beispielsweise poröse, natürlich vorkommende oder synthetische Polymere, Glas, keramische Materialien, Zellulosematerialien. Die Materialien können beispielsweise aus Filmen, Fasern oder Partikeln (beispielsweise Kügelchen, die durch Kleb- oder Bindematerialien zusammengehalten werden) zu einer Membran gefertigt werden. Besonders bevorzugt besteht die Membran aus Polycarbonat, Nitrozellulose, Zelluloseactetat, Polyamid, Polyester, Polyvinylidenfluorid oder Nylon. Am meisten bevorzugt besteht die Membran aus Nitrozellulose. Der Ausdruck "Nitrozellulose" bezieht sich auf Ester der Zellulose mit Nitrat-Gruppierungen. Der Ausdruck "Nitrozellulose" umfasst zudem Ester der Zellulose mit Nitrat-Gruppierungen alleine oder mit einem Gemisch aus verschiedenen Estern, die durch andere Säuren als die Nitriersäure erzeugt wurden, insbesondere aliphatische carbocyclische Säuren.

Die flüssigkeitsdurchlässige Membran kann auf jede geeignete Art und Weise in der Kartusche befestigt werden. Beispielsweise kann die Membran durch Heißklebung, sonicwelding (Schallschweißen), Schweißen, Kleben oder Einpressen in der Kartusche befestigt werden. Bevorzugt ist die Membran in die Kartusche eingeschweißt.

Für die Durchführung der Multiparameter-Nachweisreaktionen sind auf der Oberfläche der flüssigkeitsdurchlässigen Membran Sonden angebracht. Der Ausdruck "angebracht" bedeutet, dass diese Substanzen eine ausreichende Affinität zu der Membran aufweisen oder mit dieser chemisch so verbunden sind, dass das Aufbringen der Probenflüssigkeit bzw. der eventuell weiter notwendigen Wasch- und Reaktionslösungen nicht zur Ablösung dieser Verbindungen führt.

Zur Durchführung der erfindungsgemäßen Multiparameter-Nachweisreaktionen sind daher alle Sonden geeignet, die entweder direkt oder durch Linker an die Membran angebracht werden können. Der Ausdruck "Linker" bedeutet hierbei, dass eine Sonde unter Verwendung einer bekannten Technik mit einem Linker chemisch verbunden wird und der Linker dann eine besser Haftung (durch kovalente, chemische Bindung oder andere Wechselwirkungen) an der Membranoberfläche ermöglicht. Beispielsweise ist das Avidin/Streptavidin-System zu diesem Zweck geeignet. Durch die Verwendung von Linkern werden die Antigene den in der Reaktionslösung vorhandenen Antikörpern meist besser präsentiert.

Des Weiteren müssen die Sonden zum Nachweis eines Parameters geeignet sein. Parameter können durch Substanzen, wie beispielsweise Antikörper, Antigene oder Nukleinsäuren, die in der Probenflüssigkeit vorkommen, in quantitativer oder qualitativer Weise nachgewiesen bzw. bestimmt werden. So stellt der Nachweis eines Krankheitserregers eine Bestimmung eines Parameters dar, wobei die Krankheitserreger beispielsweise über ihre Antigene nachgewiesen werden können. Um einen Parameter nachzuweisen, muss sich ein Komplex aus der Sonde und der dem nachzuweisenden Parameter zugrundeliegenden Substanz (z.B. Antigen) bilden, wobei der Ausdruck "Komplex" jede geeignete Wechselwirkung zwischen der Sonde und der Substanz umfasst.

In der erfindungsgemäßen Vorrichtung können unterschiedliche Sonden zur Bestimmung von verschiedenen Parametern auf der Oberfläche der flüssigkeitsdurchlässigen Membran angebracht werden. Der Ausdruck "Sonden" umfasst jegliche Reagenzien und Moleküle zum Nachweis von diagnostisch interessanten Parametern. Die Sonden können beispielsweise aus Tieren oder Menschen isoliert werden oder synthetisch erzeugt werden. Bevorzugte Sonden sind Antikörper, Antigene und Nukleinsäuren. Chemisch unterschiedliche Sonden gehören zu unterschiedlichen "Arten" von Sonden, beispielsweise stellen verschiedene Antikörper unterschiedliche Arten von Sonden dar. Jedoch können unterschiedliche Arten von Sonden für den Nachweis desselben Krankheitserreger geeignet sein.

Besonders bevorzugte Sonden sind spezifische Antikörper für den Nachweis von Krankheitserregern, ausgewählt aus der Gruppe bestehend aus: HIV (Humanes Immundefizienz-Virus), HCV (Hepatitis C-Virus), PAV (Parvovirus), EBV (Epstein-Barr-Virus), CMV (Cytomegalovirus), HSV (Herpes Simplex-Virus), VZV (Varicella-Zoster-Virus), *B. burgdorferi (Borrelia burgdorferi*), *T. gondii (Toxoplasma gondii*), *T. pallidum (Treponema pallidum*), *C. jejuni (Campylobacter jejum*), *H. pylori (Heliobacter pylori*), *Y. enterocolitica (Yersinia enterocolitica).*

Bevorzugt werden die unterschiedlichen Sonden, die auf der flüssigkeitsdurchlässigen Membran angebracht werden, derart ausgewählt, dass sie zur Abklärung einer bestimmten Symptomatik geeignet sind. Weiter bevorzugt werden die Sonden für die Abklärung einer Symptomatik, ausgewählt aus einer Gruppe bestehend aus: unklarer Symptomatik (bevorzugt bei einem Zustand mit unklarem Fieber, Lymphknotenschwellung, generellem Unwohlsein), rheumatischen/arthritischen Beschwerden, respiratorischen Infekten und mykobakteriellen Infektionen. Geeignete Sonden für die Abklärung einer unklaren Symptomatik (bevorzugt bei einem Zustand mit unklarem Fieber, Lymphknotenschwellung, generellem Unwohlsein) sind Antigene gegen Krankheitserreger, ausgewählt aus der Gruppe bestehend aus: EBV, HSV, CMV, VCV, Parvovirus B19, Borrelia burgdorferi und Treponema pallidum. Für die Abklärung von rheumatischen/arthritischen Beschwerden geeignete Sonden sind Antigene gegen Krankheitserreger, ausgewählt aus der Gruppe bestehend aus: Parvovirus B19, CMV, EBV, Yersinia enterocolitica, Campylobacter und Antigene aus der Autoimmundiagnostik (ANA/ENA), bevorzugterweise Parvovirus B19, CMV, EBV, Yersinia enterocolitica, und Campylobacter. Für die Abklärung der Symptomatik eines respiratorischen Infekts geeignete Sonden können durch Verwendung von Nukleinsäuresonden nachgewiesen werden, die aus der Erbinformation der Krankheitserreger durch Amplifikation der DNA/RNA mit verschiedenen Primern und unter Verwendung des PCR-Verfahrens hergestellt werden können.

Das Anbringen der unterschiedlichen Sonden auf der Membranoberfläche kann nach bekannten Verfahren erfolgen. Verfahren zum Immobilisieren der Sonden auf der Membranoberfläche sind bekannt. Beispielsweise können die Sonden mit einem Spot-Verfahren auf die flüssigkeitsdurchlässige Membran aufgetragen werden. Bevorzugterweise wird eine Art von Sonde in punktförmiger Weise aufgetragen, wobei die Punkte mit den verschiedenen Arten auf der flüssigkeitsdurchlässigen Membran räumlich getrennt voneinander sind. Das Anbringen der Sonden an die flüssigkeitsdurchlässige Membran bzw. deren Oberfläche kann mittels einer dünnen Nadel erfolgen, die nach Eintauchen in die Sonden-Lösung (Antigen/Oligonukleotid) eine geringe Flüssigkeitsmenge aufnimmt und nach Absetzen auf der Membran diese wieder abgibt und damit die Teststelle ("Spot") erzeugt. Eine andere Möglichkeit stellt das berührungslose Aufbringen der Sonden durch Abgabe kleiner Flüssigkeitstropfen aus einer Düse, unmittelbar über der jeweiligen Stelle auf der Membran, dar.

Zusätzlich ist es bevorzugt, mehrere Arten von Sonden für dieselbe Krankheit auf die flüssigkeitsdurchlässige Membran, räumlich getrennt voneinander aufzutragen, um beispielsweise eine Differenzierung des Infektionsablaufs oder des Serotyps zu erreichen. Besonders bevorzugt werden mehrere Sonden wie beispielsweise Antigene für Krankheiten verwendet, ausgewählt aus der Gruppe bestehend aus: Epstein-Barr-Virus (EBV), Cytomegalovirus (CMV), Herpes Simplex-Virus (HSV), sexuell übertragbaren Krankheiten (STD), Pneumonien und Autoimmunkrankheiten, bevorzugt Epstein-Barr-Virus (EBV), Cytomegalovirus (CMV) und Herpes Simplex-Virus (HSV).

Zusätzlich ist bevorzugt dass mindestens 4, weiter bevorzugt 8, noch weiter bevorzugt 12, weiter bevorzugt 30, noch weiter bevorzugt 50 und noch weiter bevorzugt 100 verschiedene Arten von Sonden auf der flüssigkeitsdurchlässigen Membran angebracht sind. Weiter bevorzugt enthält die Membranoberfläche Teststellen für Kontrollzwecke wie z.B. Färbekontrollen für korrekten Testablauf, Anzeige des verwendeten Konjugats oder cutoff-Kontrollen für die Berechnung der relativen Intensität der gefärbten Teststellen.

Zusätzlich können die unterschiedlichen Sonden als Gemisch auf der Membranoberfläche angebracht werden, falls eine selektive Detektierung des Komplexes aus der Sonde und der zum Parameter zugehörigen Substanz wie beispielsweise ein Antigen, beispielsweise durch Detektion bei unterschiedlichen Wellenlängen, möglich ist.

Bevorzugterweise werden die unterschiedlichen Arten von Sonden räumlich getrennt voneinander auf die Oberfläche der flüssigkeitsdurchlässigen Membran angebracht. Die Stellen mit jeweils der gleichen Art von Sonde können dann räumlich getrennt von den anderen Stellen mit den weiteren Arten getrennt sein. Diese Vorgehensweise ermöglicht die Zuordnung von positiven oder negativen Farbreaktionen zu den spezifischen Sonden.

Eine visuelle oder automatisierte Auswertung, beispielsweise mittels einem Scanner der Testergebnisse wird dadurch möglich. Bei visueller Auswertung der Testergebnisse ist es zusätzlich bevorzugt, eine Schablone zu verwenden, die nach Entfernen der Kamine auf die Kartusche aufgelegt werden kann. Diese Schablone kann beispielsweise aus Papier bestehen und Löcher an den Stellen enthalten, an denen die Sonden aufgetragen worden sind. Eine Beschriftung neben diesen Löchern kann dann die Auswertung, ob eine Farbreaktion aufgetreten ist oder nicht, erleichtern.

Für das erfindungsgemäße Verfahren und die erfindungsgemäße Vorrichtung können jegliche Proben, seien es Proben von Patienten oder wissenschaftliche Proben, verwendet werden. Die Proben können beispielsweise Seren von Patienten sein und unbehandelt oder behandelt (bevorzugt werden die Proben zwischen 1:40 und 1:400 in Waschpuffer verdünnt) eingesetzt werden. Bevorzugt werden ältere, gelagerte Seren nach Verdünnung, unter Verwendung eines 0,2 µm Filters, von partikulären Bestandteilen gereinigt. Bevorzugt werden die Seren von Patienten verdünnt eingesetzt.

Zum Nachweis von Komplexen aus Sonden- und den Substanzen, die dem Parameter zugrundeliegen, können bereits bekannte Verfahren verwendet werden.

Die für das erfindungsgemäße Verfahren geeigneten Wasch- und Reaktionslösungen sind dem Fachmann bekannt. Waschlösungen können beispielsweise Pufferlösungen sein, bevorzugterweise PBS (Phosphat-gepufferte Saline)-Puffer. Reaktionslösungen sind beispielsweise Lösungen für die Detektion eines Komplexes aus der Sonde und der zum Parameter zugehörigen Substanz durch Färbereaktionen und umfassen Färbereagenzien. Weitere Reaktionslösungen enthalten beispielsweise Konjugate wie Peroxidase-Konjugat und sind dem Fachmann bekannt.

Das erfindungsgemäße Verfahren zur parallelen Durchführung von Multiparameter-Nachweisreaktionen kann mit allen geeigneten Vorrichtungen und besonders bevorzugt mit allen oben beschriebenen Ausführungsformen der erfindungsgemäßen Vorrichtung durchgeführt werden. Das Verfahren umfasst mehrere Schritte und wird im Nachfolgenden näher erläutert. Hierbei sind die speziellen Methoden beispielsweise aus Immunoassays bekannt und werden nicht im Detail beschrieben. Geeignete Bindungsassays sind aus der Literatur bekannt. Das erfindungsgemäße Verfahren wird daher durch die nachfolgende Beschreibung des Verfahrens nicht auf die Anzahl der einzelnen Verfahrensschritte, die Abfolge der Schritte oder die verwendeten Reagenzien eingeschränkt.

In einem ersten Schritt wird eine Kartusche mit einer darin befestigten flüssigkeitsdurchlässigen Membran in einer Kartuschenhalterung befestigt. Durch diese Maßnahme wird auf der Unterseite der flüssigkeitsdurchlässigen Membran bzw. der Kartusche ein unteres Kompartiment erzeugt. Hierbei wird eine Membran verwendet, auf deren Oberfläche bereits immobilisierte Sonden angebracht sind.

In einem weiteren Schritt wird ein Kamin zur Erzeugung eines nach oben offenen Kompartiments auf die Kartusche aufgesetzt. Hierdurch wird eine flüssigkeitsundurchlässige Verbindung zwischen dem Kamin und der Kartusche erzeugt.

In einem weiteren Schritt wird das obere Kompartiment mit der Probenflüssigkeit befüllt. Von diesem Zeitpunkt an wird die Inkubationszeit mit den Sonden an der Membranoberfläche berechnet. Die Inkubationszeit, bevor die Probenflüssigkeit durch die flüssigkeitsdurchlässige Membran gesaugt wird, orientiert sich an den jeweiligen Nachweisreaktionen. Bevorzugt beträgt die Inkubationszeit jedoch weniger als 30, weiter bevorzugt weniger als 20 und am meisten bevorzugt weniger als 15 Minuten.

Als nächstes wird ein Unterdruck mittels einer Pumpe an das untere Kompartiment angelegt und die Probenflüssigkeit durch die flüssigkeitsdurchlässige Membran durchgesaugt. Bevorzugterweise wird die Probenflüssigkeit mit einer Geschwindigkeit von 0,1 bis 2 ml/min durch die flüssigkeitsdurchlässige Membran gesaugt. Bei diesem Schritt ist es möglich, dass die Pumpe bereits vor, oder zu Beginn, bzw. während oder nach der Inkubationszeit eingeschaltet wird. Bevorzugterweise wird die Pumpe nach der Inkubationszeit eingeschaltet.

Sobald die Probenflüssigkeit vollständig durch die flüssigkeitsdurchlässige Membran durchgeflossen ist, erfolgt mindestens ein Waschschritt. Geeignete Waschlösungen sind dem Fachmann bekannt. Die Fließgeschwindigkeit, mit der die Waschlösung durch die flüssigkeitsdurchlässige Membran gesaugt wird, kann hierbei größer sein als die Fließgeschwindigkeit beim Durchsaugen der Probelösung. Hierbei ist es besonders bevorzugt, dass die Geschwindigkeit, mit der die Waschlösung durch die flüssigkeitsdurchlässige Membran gesaugt wird, 2 bis 10 ml/min, weiter bevorzugt 3 bis 5 ml/min beträgt.

Nach eventuellen weiteren Waschschritten wird Reaktionslösung in das obere Kompartiment gefüllt und durch die flüssigkeitsdurchlässige Membran gesaugt, um eine Farbreaktion der Komplexe aus Sonde und der dem Parameter zugrundeliegenden Substanz zu vermitteln. Bevorzugt wird zunächst eine Lösung mit Konjugat und anschließend eine Lösung zur Vermittlung einer Farbreaktion verwendet.

In einem weiteren Schritt werden die Kamine der Kartuschen entfernt.

In einem anschließenden Schritt werden die Testergebnisse visuell, bspw. mit einer Referenzschablone oder automatisch mit einem Scanner ausgelesen und die erzeugten farbigen Stellen bzw. die farblos gebliebenen Stellen ausgewertet.

Die Multiparameter-Nachweisreaktionen erzeugen farbige Stellen auf der Membranoberfläche. Diese farbigen Stellen können visuell oder durch technische Detektionsverfahren erfasst und den jeweiligen Sonden zugeordnet werden. Eine positive Farbreaktion deutet hierbei den Nachweis der speziellen Parameter an, wobei eine Stelle mit Sonden, die sich nicht verfärbt hat, die Abwesenheit dieses Parameters anzeigt. Eine automatisierte Detektion der farbigen Stellen auf der Membranoberfläche ist mittels eines Scanners im sichtbaren Bereich oder mit anderen Absorptionsspektrometern möglich. Besonders bevorzugt sind die unterschiedlichen Komplexe aus Sonde und der dem Parameter zugrundeliegenden Substanz bei unterschiedlichen Wellenlängen mittels eines Absorptionsspektrometers detektierbar. Die räumlich getrennte Anordnung der Sonden ermöglicht jedoch auch die Detektion von Komplexen gleicher Färbung.

### Erläuterung der Figuren

**Figur 1****:** Untersuchung der Serum-Reaktivitäten von 6 Seren mit kommerziell erhältlichen Testsystemen *recom*Line, *recom*Blot oder *recom*Well (Firma Mikrogen).
   Verwendete Abkürzungen: *B. burgdorferi* (Borrelia burgdorferi), HIV (Humanes Immundefizienz-Virus), HCV (Hepatitis C-Virus), PAV (Parvovirus), EBV (Epstein-Barr-Virus), CMV (Cytomegalovirus), HSV (Herpes simplex-Virus), VZV (Varizella Zooster Virus), *B. burgdorferi (Borrelia burgdorferi), T. gondii (Toxoplasma gondii), T. pallidum (Treponema pallidum), C. jejuni (Campylobacter jejuni), H. pylori (Helicobacter pylori), Y. enterocolitica (Yersinia enterocolitica), C. trachomatis (Chlamydia trachomatis), HEV (Hepatitis E-Virus).*
**Figur 2****:** Untersuchung der Serum-Reaktivität von Serum 1 mit der erfindungsgemäßen Vorrichtung (Spalte Befund) und den recom-Testsystemen (Firma Mikrogen). Das recom-Testsystem wurde als Kontrolle eingesetzt und belegt, dass mit der erfindungsgemäßen Vorrichtung zuverlässig richtige Ergebnisse erhalten werden.
**Figur 3****:** Untersuchung der Serum-Reaktivität von Serum 2 mit der erfindungsgemäßen Vorrichtung (Spalte Befund) und den recom-Testsystemen (Firma Mikrogen).
**Figur 4****:** Untersuchung der Serum-Reaktivität von Serum 3 mit der erfindungsgemäßen Vorrichtung (Spalte Befund) und den recom-Testsystemen (Firma Mikrogen).
**Figur 5****:** Untersuchung der Serum-Reaktivität von Serum 4 mit der erfindungsgemäßen Vorrichtung (Spalte Befund) und den recom-Testsystemen (Firma Mikrogen).

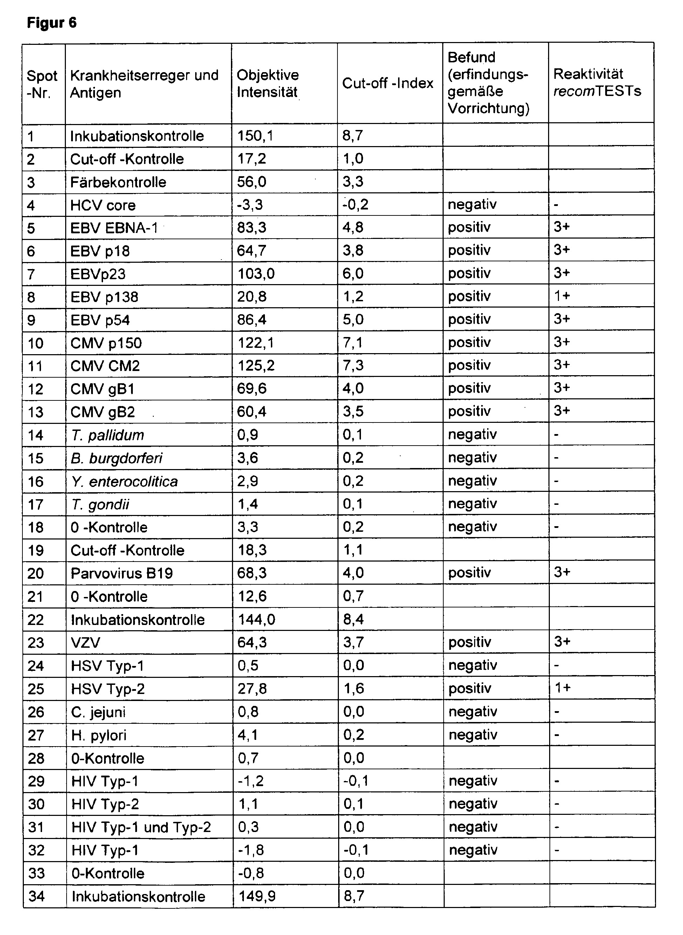

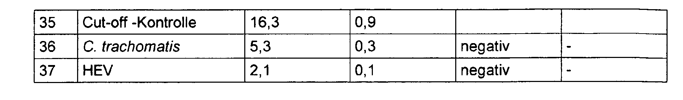

**Figur 6****:** Untersuchung der Serum-Reaktivität von Serum 5 mit der erfindungsgemäßen Vorrichtung (Spalte Befund) und den recom-Testsystemen (Firma Mikrogen

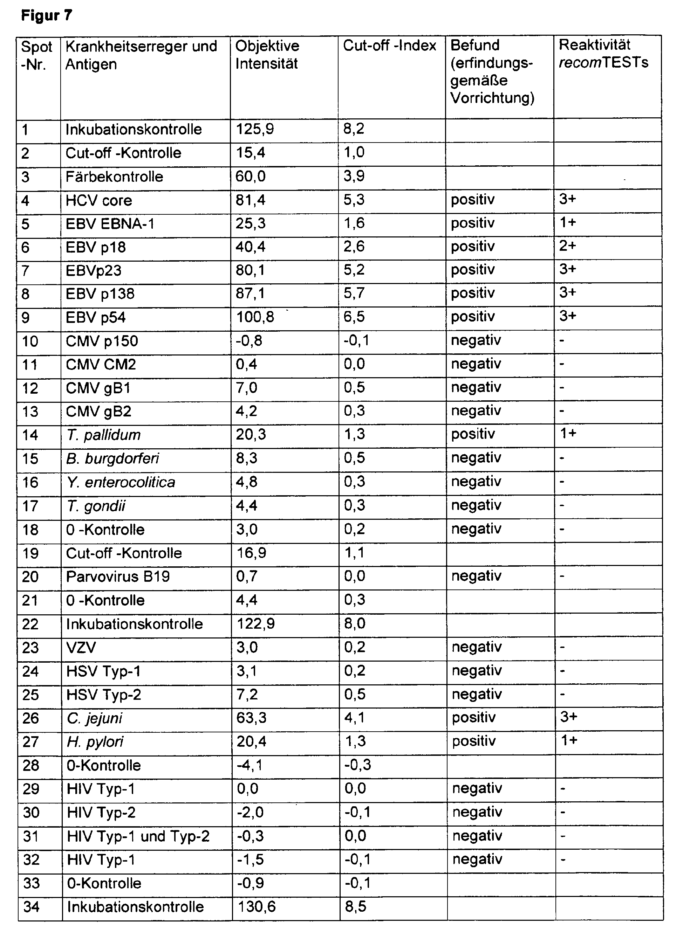

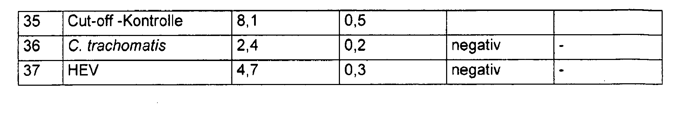

**Figur 7****:** Untersuchung der Serum-Reaktivität von Serum 6 mit der erfindungsgemäßen Vorrichtung (Spalte Befund) und den recom-Testsystemen (Firma Mikrogen).

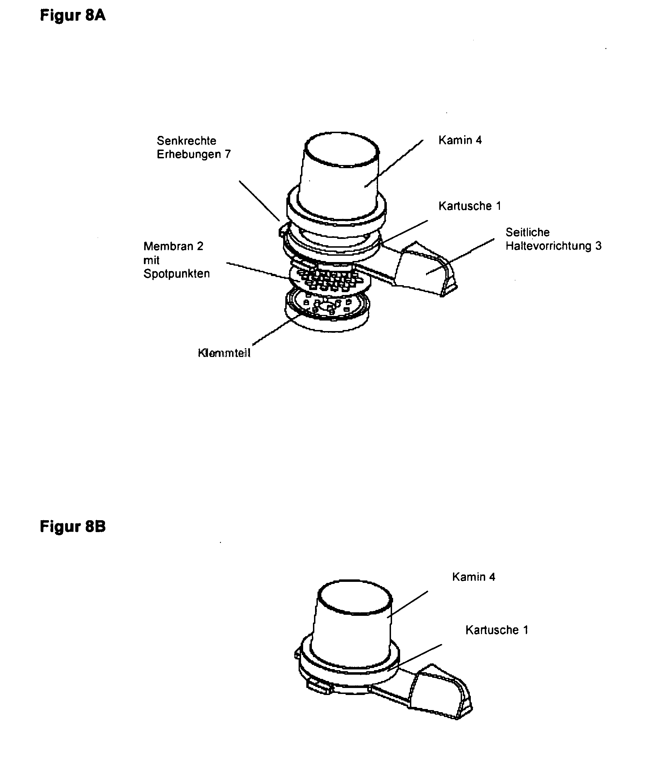

**Figur 8A** zeigt einen Ausschnitt des schematischen Aufbaus der erfindungsgemäßen Vorrichtung zur Durchführung von Multiparameter-Nachweisreaktionen. Figur 8A zeigt eine Kartusche 1, in der eine flüssigkeitsdurchlässige Membran 2 befestigt werden kann. Die Kartusche 1 weist des Weiteren eine seitliche Haltevorrichtung 3 auf. Auf die Kartusche 1 wird ein Kamin 4 aufgesteckt, so dass eine flüssigkeitsundurchlässige Verbindung zwischen Kamin 4 und Kartusche 1 entsteht. Des Weiteren sind zur Kartusche senkrechte (flügelförmige) Erhebungen 7 zum Befestigen in der Kartuschenhalterung 5 zu erkennen. Diese senkrechten Erhebungen 7 ermöglichen das Befestigen in der Kartuschenhalterung 5 nach Art eines Bajonettdrehverschlusses. **Figur 8B** zeigt die zusammengebaute Vorrichtung aus Kamin 4, Kartusche 1 und Membran 2.

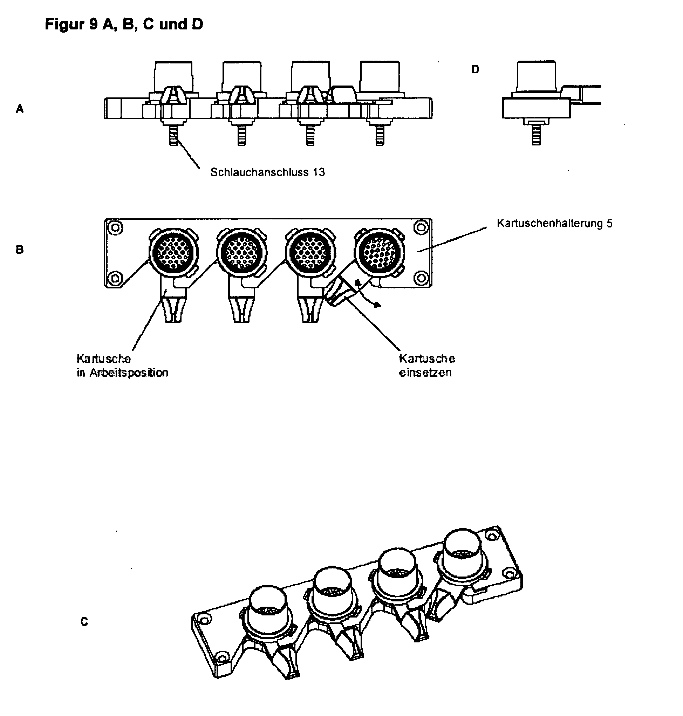

**Figuren 9A, 9B, 9C und 9D** zeigen einen Ausschnitt (verschiedene Ansichten) der erfindungsgemäßen Kartuschenhalterung 5, in der die erfindungsgemäßen Kartuschen 1 befestigt werden. **Figur 9B** zeigt die Befestigung der Kartusche 1 mit Kamin 4 in der Kartuschenhalterung 5. Die Kartusche 1 wird durch Drehbewegung in die Kartuschenhalterung 5 eingebracht.

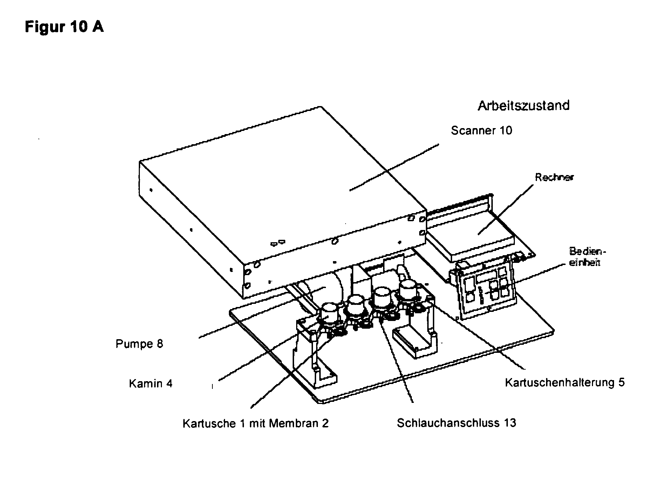

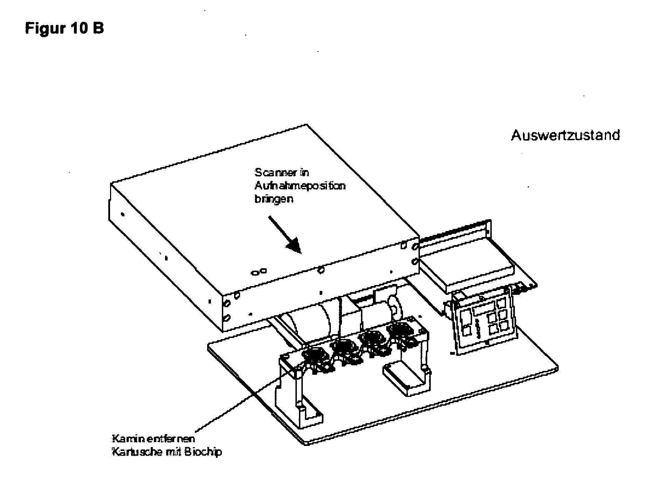

**Figur 10A** zeigt eine Gesamtansicht der erfindungsgemäßen Vorrichtung im Arbeitszustand. Hierbei ist zu erkennen, dass die Kartuschenhalterung 5 einen Schlauchanschluss 13 umfasst, der das Absaugen der Probenflüssigkeit aus dem unteren Kompartiment mittels einer Pumpe 8 in einen Entsorgungsbehälter 9 (nicht dargestellt) ermöglicht. Die Kartuschenhalterung 5 ist auf die Trägerplatte 12 montiert. Des Weiteren ist ein integrierter Scanner 10 dargestellt, der beweglich montiert ist und nach Abnehmen des Kamins 4 von der Kartusche 1 senkrecht über die flüssigkeitsdurchlässige Membran 2 bewegt werden kann, um die Testergebnisse auszuwerten. Die so ermittelten Daten werden dann an einen Computer weitergegeben. **Figur 10B** zeigt eine Gesamtansicht der erfindungsgemäßen Vorrichtung im Auswertzustand nach Abnahme der Kamine 4 von der Kartusche 1. Der Pfeil deutet die Bewegung des Scanners 10 an, der über die flüssigkeitsdurchlässigen Membranen 2 geschoben werden kann um die Testergebnisse auslesen zu können.

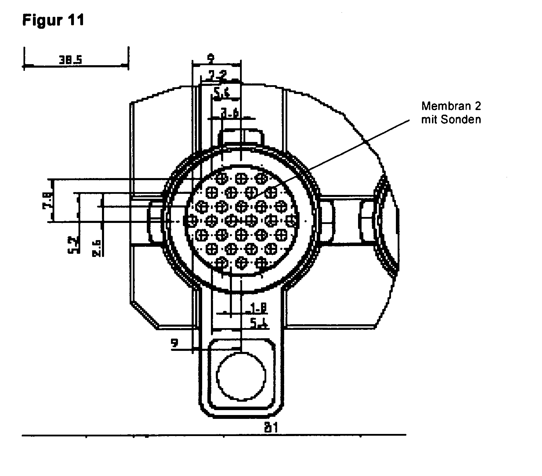

**Figur 11** zeigt eine erfindungsgemäße flüssigkeitsdurchlässige Membran 2 mit voneinander räumlich getrennten Sonden (angedeutet).

### Beispiel 1: Durchführung eines serologischen Tests zum Nachweis von EBV-Antigenen

Für den Nachweis von EBV(Epstein-Barr-Virus)-Antikörpern werden einmal-verwendbare Kartuschen mit Nitrozellulose-Membranen (Porengröße von 0,6 µm) verwendet. Die flüssigkeitsdurchlässigen Membranen tragen die rekombinanten EBV-Antigene EBNA-1, p18, p23, p54, p18L und p138. Hierbei wurden die Antigene berührungslos als kleine Tropfen mit einem Volumen von 0,16 µl aufgebracht. Für den cutoff wurde eine Lösung aus Schwein-anti-Kaninchen-lgG verwendet, welches mit dem POD-konjugierten Konjugat reagiert. Jede Membran enthält drei Cutoff-Spots pro Membran (Position 2,19 und 35), wobei der Cutoff als Mittelwert aus den drei einzelnen Cutoff-Spots berechnet würde. Die Kartuschen werden mit einem Kamin versehen und in die Kartuschenhalterung eingebracht.

Das zu testende Patientenserum wird verdünnt (1:40) und anschließend durch einen 0,22 µm-Filter geklärt, um ein Verstopfen der Membranporen zu verhindern. Die Verdünnung und alle Waschschritte erfolgen in einem PBS (Phosphat-gepufferte Saline)-Puffer. 2 ml verdünntes Patientenserum werden in die Kartusche gefüllt und kontinuierlich durch die flüssigkeitsdurchlässige Membran gesaugt. Anschließend folgen 3 kurze Waschschritte mit je 2 ml PBS-Puffer. Zum Nachweis der an die Antigene gebundenen humanen Antikörper wird 2 ml Peroxidase-konjugierte Anti-Human-IgG-Kaninchenantikörper (Verdünnung 1:400) zugegeben. Gebundene Kaninchenantikörper werden anschließend nach 3 weiteren kurzen Waschschritten mittels TMB (Tetramethylbenzidin) oder DAB (Diaminobenzidin) nachgewiesen. Die Farbreaktion wird durch einen anschließenden Waschschritt mit Wasser gestoppt. Bei den Inkubationsschritten werden alle Lösungen mit einer Geschwindigkeit von 0,5 ml/min durch die flüssigkeitsdurchlässige Membran gesaugt. Waschschritte erfolgen mit 4-facher Fließgeschwindigkeit. Die Auswertung eines Tests kann mit dem Auge erfolgen. Darüber hinaus besteht die Möglichkeit, die entwickelten Membranen einzuscannen und mit einem Bildbearbeitungssystem auszuwerten. Die Beurteilung und Bewertung der Antigenspots wird mit Hilfe eines auf der Membran befindlichen Cut-Offs durchgeführt.

### Beispiel 2: Nachweis Staphylokokken-spezifischer Nukleinsäuren aus Patientenproben

MRSA-Infektionen werden weltweit immer häufiger beobachtet. Die schnelle Differenzierung von *Staphylococcus aureus, S. epidermidis* und *S. saprophyticus* sowie die Detektion der möglichen Resistenzmutationen bei *S. aureus* könnte dabei helfen, die Verbeitung dieser Keime einzudämmen.

Ein besonderes Problem stellt dieser Keim in Krankenhäuser dar, da durch eine unbeabsichtigte Einschleppung über neu aufgenommene Personen andere Patienten mit oft geschwächten Immunstatus infiziert werden können und damit neben der Grunderkrankung weitere massive Komplikationen auftreten.

Als Konsequenz daraus müssten alle Patienten mit MRSA-Risiko bei Aufnahme bis zur Abklärung einer möglichen MRSA-Belastung isoliert und getestet werden. Da Isoliermaßnahmen in den Kliniken sehr teuer sind, besteht ein großes Interesse daran, möglichst schnell eine MRSA-Gefährdung abzuklären.

Dazu werden DNA-Nachweis-Sonden (Oligos mit 17-19 Basenpaaren), die erregerspezifische Bereiche aus dem Genom von Bakterien repräsentieren, mit 200pMol pro Teststelle auf eine Nitrozellulose-Membran (Porengröße 0,6µm) in der Trägervorrichtung gespottet (vgl. Beispiel 1) und durch UV-Bestrahlung verankert.

Das Probenmaterial (markierte Nukleinsäure-Amplifikationsprodukte oder genomische DNA) wird denaturiert, mit 0,5 ml Hybridisierungspuffer (0,1% NLS (N-Lauroyl-Sarcosin Na-Salz), 0,02% SDS, 5x SSC: 0,075M Trinatriumcitrat, 0,75M NaCl) gemischt und in die Kartusche gefüllt. Die Probe wird 15 min bei RT inkubiert und dann durch die flüssigkeitsdurchlässige Membran gesaugt (Geschwindigkeit von 0,4 ml/min). Es folgen ein langer (2 min Inkubation) und zwei kurze (keine Inkubation) Waschschritte mit auf 52°C temperierter stringenter Waschlösung (0,1 % SDS, 2x SSC: 0,03 M Trinatriumcitrat, 0,3 M NaCl) sowie ein kurzer Waschschritt mit Waschpuffer (0,05 M Na₂HPO₄, 0,0085 M NaH₂PO₄, 1,4 M NaCl, 0,5% Tween20) bei Raumtemperatur. Waschlösung und -puffer werden auch mit 0,4 ml/min durchgesaugt. Der Nachweis markierter Amplifikationsprodukte erfolgt mit 1ml Peroxidase-konjugiertem Antikörper, der gegen die Markierung des Amplifikationsproduktes gerichtet ist (Verdünnung 1:400 in Waschpuffer, Inkubation 20min). Die gebundenen Antikörper werden nach drei weiteren kurzen Waschschritten mit Waschpuffer mittels Farbreaktion mit TMB nachgewiesen. Die Farbreaktion wird mit einem anschließenden Waschschritt mit Wasser gestoppt. Die Beurteilung und Auswertung der Spots erfolgt per Auge anhand der Kontrollen auf der Membran. Darüber hinaus besteht die Möglichkeit, die entwickelten Membranen einzuscannen und mit einem Bildbearbeitungsprogramm auszuwerten.

### Beispiel 3. Nachweis von 13 verschiedenen Krankheitserregern

Die erfindungsgemäße Vorrichtung wird in diesem Beispiel für einen simultanen Nachweis von spezifischen Antikörpern gegen die 13 häufigsten Krankheitserreger in 15 min verwendet. Dazu wurden zunächst 4 Seren mit käuflichen Testsystemen *recom*Line (Firma Mikrogen), *recom*Blot (Firma Mikrogen) oder *recom*Well (Firma Mikrogen) getestet, um Vergleichsdaten zu erhalten. Um eine Differenzierung des Infektionsablaufs oder des Serotyps zu erreichen, wurden bei manchen Krankheitserregern (EBV, CMV und HSV) mehrere Antigene räumlich getrennt aufgetragen (Spot-Nr. 5-15, 26 und 27).

Anschließend wurde die flüssigkeitsdurchlässige Membran mit den folgenden Sonden und Kontrollreagenzien bespottet:

| | | | | | |
|---|---|---|---|---|---|
| **1.** | Inkubationskontrolle | **13.** | PAV-Partikel | **23.** | *B. burgdorferi* |
| **2.** | Cut-Off-Kontrolle | **14.** | PAV VP-N | **24.** | *T. gondii* |
| **3.** | Färbekontrolle l | **15.** | PAV VP_C | **25.** | VZV-Partike |
| **4.** | HCV | **16.** | O-Kontrolle | **26.** | HSV-1 |
| **5.** | EBV EBNA-1 | **17.** | O-Kontrolle | **27.** | HSV-2 |
| **6.** | EBV p18 | **18.** | O-Kontrolle | **28.** | *H. pylori* |
| **7.** | EBV p23 | **19.** | Cut-Off-Kontrolle | **29.** | O-Kontrolle |
| **8.** | EBV p138 | **20.** | *T. pallidum* | **30.** | O-Kontrolle |
| **9.** | EBV p54 | **21.** | O-Kontrolle | **31.** | HIV |
| ***10.*** | CMV p150 | **22.** | Inkubationskontrolle | **32.** | *C*. *jejuni* |
| ***11.*** | CMV CM3 | | | **33.** | *Y. enterocolitica* |
| **12.** | CMV gB1 | | | **34.** | Inkubationskontrolle |
| | | | | **35.** | Cut-Off-Kontrolle |
| | | | | **36.** | O-Kontrolle |
| | | | | **37.** | O-Kontrolle |

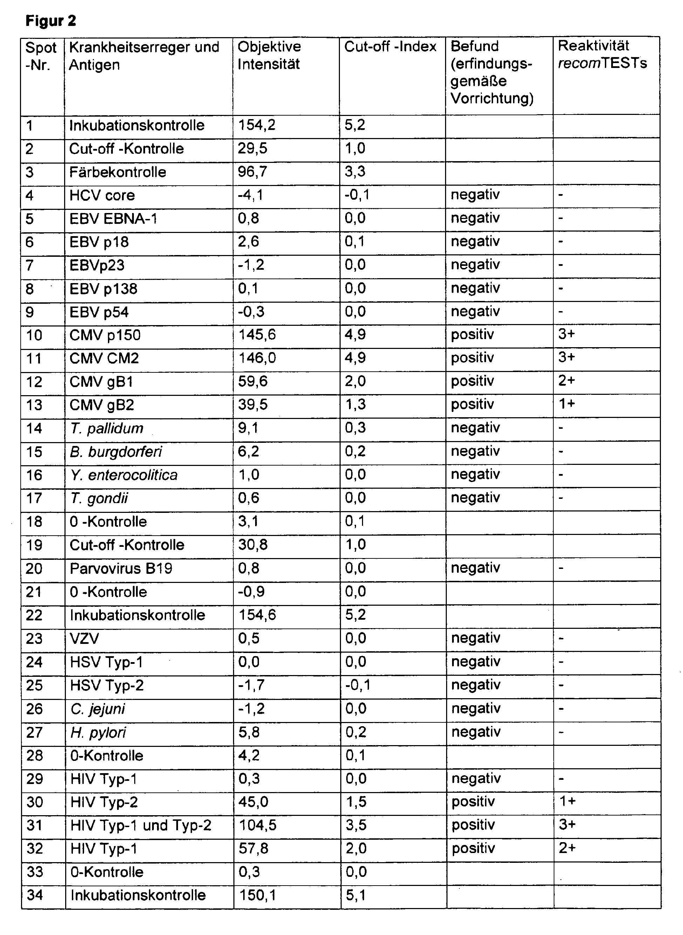

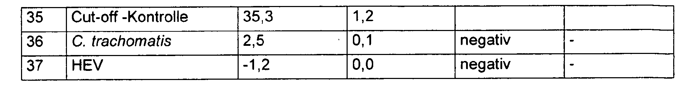

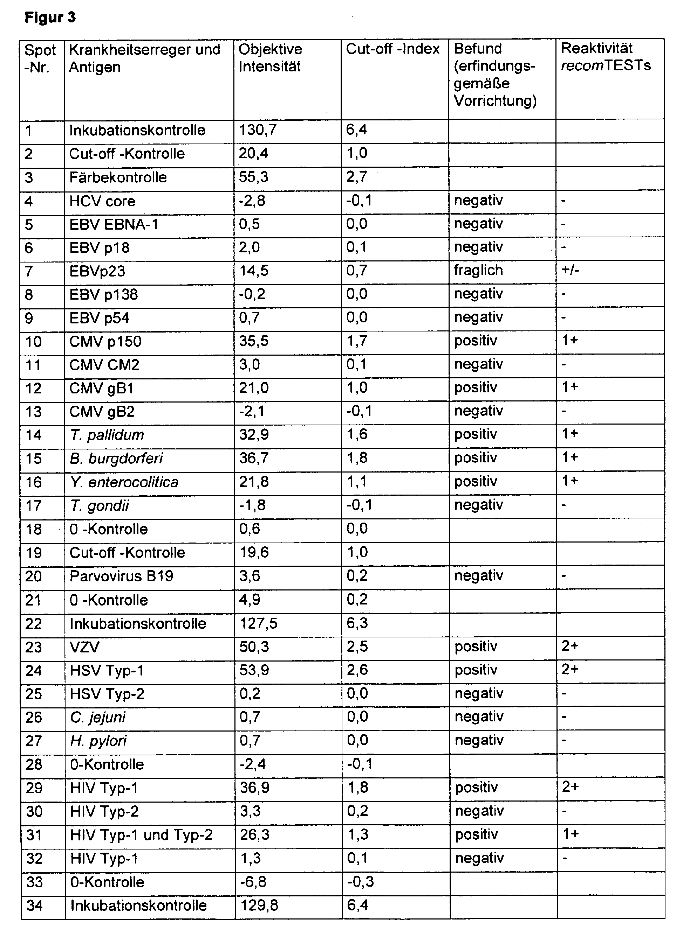

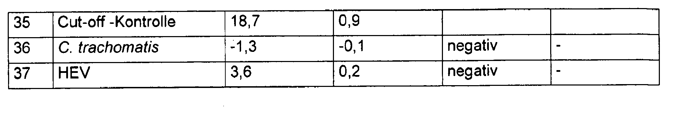

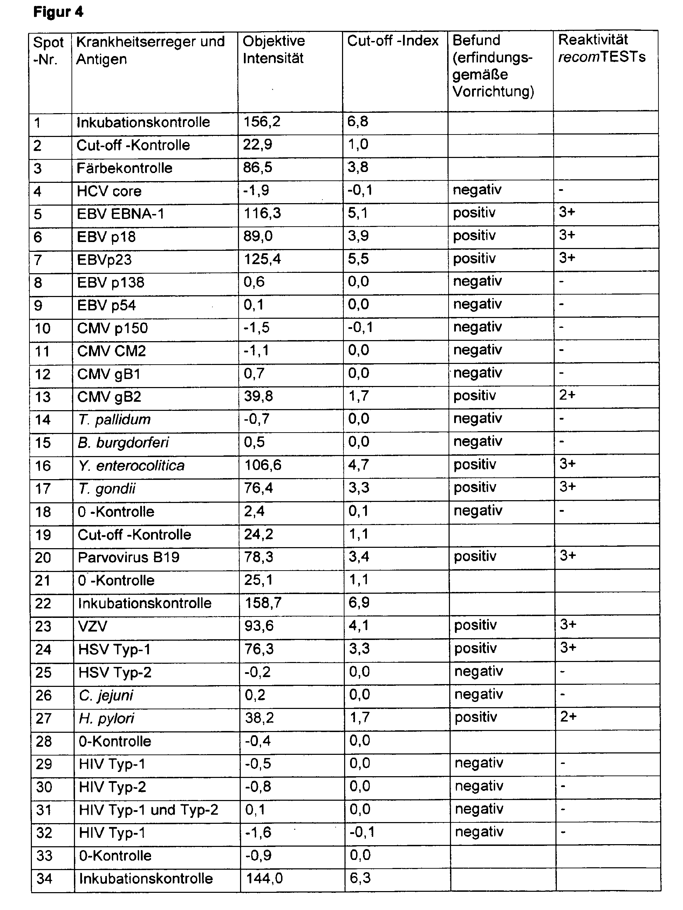

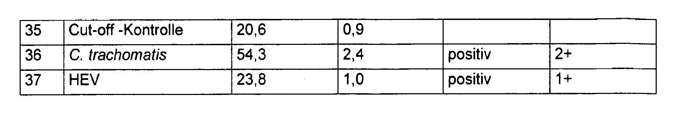

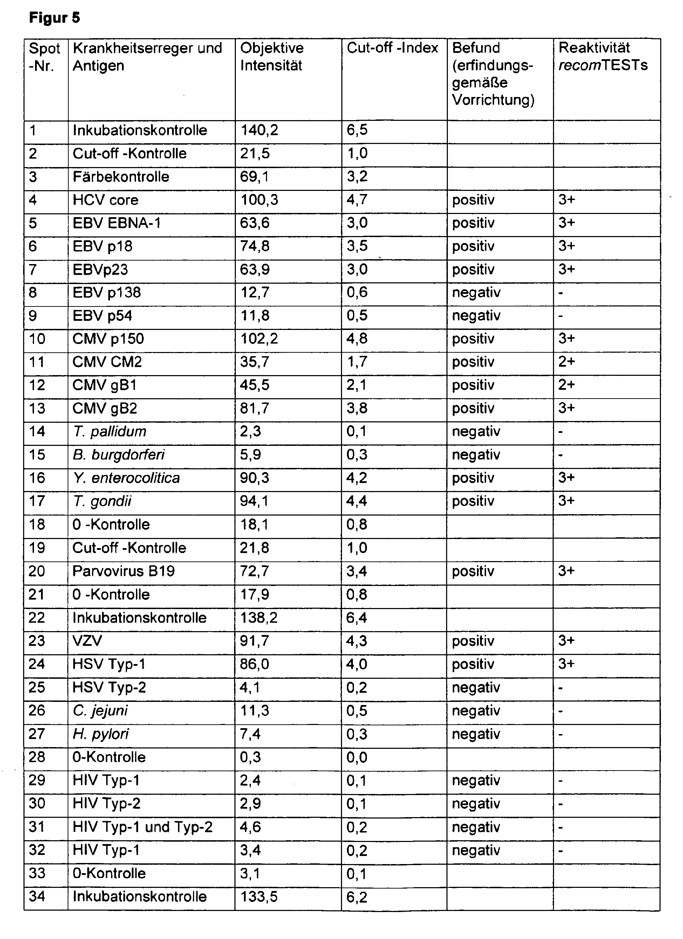

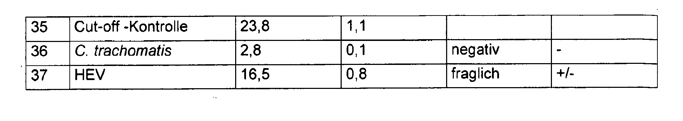

Die Testergebnisse mit den bekannten Testsystemen sind in Figur 1 dargestellt. Die Testergebnisse, die mit der erfindungsgemäßen Vorrichtung erhalten wurden, sind in den Figuren 2-5 dargestellt.

## Patentansprüche

1. Vorrichtung zur parallelen Durchführung von Multiparameter-Nachweisreaktionen an mehreren Proben, umfassend:
a) mindestens zwei Kartuschen (1),
b) je Kartusche (1) eine darin befestigte, flüssigkeitsdurchlässige Membran (2), worin die flüssigkeitsdurchlässige Membran (2) an der unteren Seite verstärkt ist,
c) einen Kamin (4), der auf der Kartusche so befestigt ist, dass ein nach oben offenes, oberes Kompartiment erzeugt wird, und
d) auf der ins obere Kompartiment weisenden, oberen Oberfläche der flüssigkeitsdurchlässigen Membran (2) angebrachte Sonden, die den gleichzeitigen Nachweis von verschiedenen Parametern in einer Probe ermöglichen,
e) eine Kartuschenhalterung (5) für die Kartuschen (1), welche das Absaugen von Flüssigkeiten aus dem oberen Kompartiment durch die flüssigkeitsdurchlässige Membran (2) ermöglicht, und
f) Mittel zum Erzeugen eines Vakuums an der Unterseite der flüssigkeitsdurchlässigen Membran (2).

2. Vorrichtung nach Anspruch 1, worin die flüssigkeitsdurchlässige Membran (2) einen Durchmesser zwischen 1 cm und 10 cm hat.

3. Vorrichtung nach einem der vorangegangenen Ansprüche, worin die flüssigkeitsdurchlässige Membran (2) aus einem Material besteht, ausgewählt aus der Gruppe bestehend aus: Nitrozellulose, Polycarbonat, Polyamid, Polyester, Polyvinylidenfluorid und Nylon.

4. Vorrichtung nach einem der vorangegangenen Ansprüche, worin das obere Kompartiment ein Volumen von 1-8 ml aufnehmen kann.

5. Vorrichtung nach einem der vorangegangenen Ansprüche, worin die flüssigkeitsdurchlässige Membran (2) Teststellen für Kontrollzwecke aufweist, die eine Berechnung der relativen Intensitäten der gefärbten Stellen auf der Membranoberfläche nach Durchführung der Multiparameter-Nachweisreaktionen ermöglichen.

6. Vorrichtung nach einem der vorangegangenen Ansprüche, worin die Probenflüssigkeit mittels einer Pumpe (8) mit einer Fließgeschwindigkeit von 0,1-2 ml/min und die Waschlösung mit einer Fließgeschwindigkeit von 2-10 ml/min, durch die flüssigkeitsdurchlässige Membran (2) gesaugt wird.

7. Vorrichtung nach einem der vorangegangenen Ansprüche, worin die flüssigkeitsdurchlässige Membran (2) eine Porengröße von 0,2-0,8 µm aufweist.

8. Vorrichtung nach einem der vorangegangenen Ansprüche, weiterhin umfassend einen Scanner (10) zum optischen Auslesen der Testergebnisse.

9. Vorrichtung nach einem der vorangegangenen Ansprüche, worin die Kartusche (2) durch eine seitliche Drehbewegung mit der Kartuschenhalterung (5) lösbar verbunden werden kann.

10. Vorrichtung nach einem der vorangegangenen Ansprüche, worin die unterschiedlichen Sonden auf voneinander räumlich getrennten Stellen auf der flüssigkeitsdurchlässigen Membran (2) angebracht sind.

11. Vorrichtung nach einem der vorangegangenen Ansprüche, worin unterschiedliche Arten von Sonden für dieselben Krankheitserreger auf voneinander räumlich getrennten Stellen auf der flüssigkeitsdurchlässigen Membran (2) angebracht sind.

12. Vorrichtung nach einem der vorangegangenen Ansprüche, worin mindestens 4 verschiedene Arten von Sonden auf der flüssigkeitsdurchlässigen Membran (2) angebracht sind.

13. Vorrichtung nach einem der vorangegangenen Ansprüche, worin die Kartusche eine seitliche Haltevorrichtung (3) aufweist, die als Mittel zum Greifen der Kartusche dient.

14. Vorrichtung nach einem der vorangegangenen Ansprüche, worin mindestens zwei Arten von Sonden für den gleichen Krankheitserreger verwendet werden, wobei der Krankheitserreger ausgewählt ist aus der Gruppe bestehend aus: Ebstein-Barr-Virus (EBV), Cytomegalovirus (CMV) und Herpes Simplex-Virus (HSV).

15. Verfahren zur parallelen Durchführung von Multiparameter-Nachweisreaktionen an mehreren Proben, umfassend folgende Schritte:
a) Einfüllen der Probenflüssigkeit in den Kamin der Kartusche,
b) Anlegen eines Unterdrucks an die Unterseite der flüssigkeitsdurchlässigen Membran der Kartusche zum Durchsaugen der Probenflüssigkeit durch die flüssigkeitsdurchlässige Membran,
c) mindestens zwei Waschschritte, bei denen mittels Vakuum eine Waschlösung durch die flüssigkeitsdurchlässige Membran gesaugt der Spüllösung durch die Membran,
d) Abnehmen der Kamine der Kartuschen,
e) Auslesen der Testergebnisse mit einem Scanner.

16. Verfahren nach einem der vorangegangenen Ansprüche, worin das Verfahren unter Verwendung der Vorrichtung nach einem der Ansprüche 1-14 durchgeführt wird.
